(19) Europäisches Patentamt
European Patent Office
Office européen des brevets

(11) Publication number: **0 080 245 A1**

(12) # EUROPEAN PATENT APPLICATION

(21) Application number: 82201484.1

(22) Date of filing: 22.11.82

(51) Int. Cl.³: C 07 D 471/08
// (C07D471/08, 239/00, 221/00)

(30) Priority: 21.11.81 NL 8105277

(43) Date of publication of application: 01.06.83 Bulletin 83/22

(84) Designated Contracting States: AT BE CH DE FR GB IT LI NL SE

(71) Applicant: STAMICARBON B.V., Postbus 10, NL-6160 MC Geleen (NL)

(72) Inventor: Corvers, Antonius, Kelmonderstraat 33, NL-6191 RD Beek (L.) (NL)
Inventor: Hofman, Johannes H. A., Brederodestraat 6, NL-6181 BE Elsloo (L.) (NL)

(74) Representative: Pinckaers, August René et al, OCTROOIBUREAU DSM Postbus 9, NL-6160 MA Geleen (NL)

(54) **Diazabicyclo(2,2,2)octadiones and process for their preparation.**

(57) Diazabicyclo (2,2,2) octadiones having the formula:

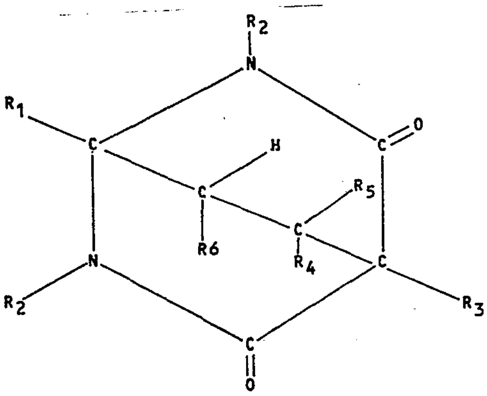

I

wherein $R_1$–$R_6$ are independently selected from the group consisting of hydrogen, methyl, and ethyl.

A method for synthesizing the compounds shown above is also disclosed and consists essentially of:

A. reacting a compound having the formula:

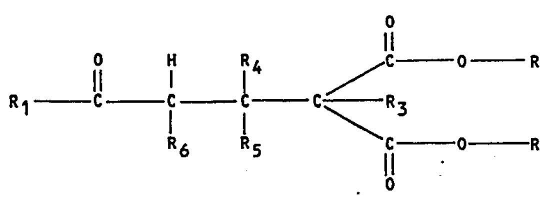

II

wherein R and R′ are independently selected from the group consisting of hydrogen and alkyl or cyclo alkyl having 1 to 10 carbon atoms, and $R_1$–$R_6$ are independently selected from the group consisting of hydrogen, methyl, and ethyl, with

B. a reactant selected from the group consisting of ammonia and primary amines having the formula $R_2$–$NH_2$ wherein $R_2$ is selected from the group consisting of hydrogen, methyl, and ethyl.

EP 0 080 245 A1

0080245



# DIAZABICYCLO(2,2,2)OCTADIONES AND PROCESS FOR THEIR PREPARATION

The invention relates to a group of new compounds of the type of diazabicyclo(2,2,2)octadione and to a process for the preparation of such compounds.

Dutch Patent Application 6400946 laid open to public inspection describes 2,5-diazabicyclo(2,2,2)octadiones as starting products for the preparation of the corresponding octanes.

These diazabicyclo(2,2,2)octanes in the N-substituted and unsubstituted forms find application as antihistamines, antiemetics, antiallergics, tranquillizers, antiparasitics, hypotensive agents, central nervous system depressants, sedatives, antibiotics, spasmolytics, analgesics, and in some cases as central nervous system stimulants and the like.

The invention provides a new group of compounds which are characterized by Formula I of the formula sheet, in which $R_1$ to $R_6$ may be the same or different and represent a hydrogen atom or a methyl or ethyl group. The compounds are substituted or unsubstituted 2,6-diazabicyclo(2,2,2)octa-3,5-diones.

According to a preferred embodiment of the invention $R_1$ is an ethyl or methyl group.

According to another preferred embodiment of the invention $R_2$ is an ethyl or methyl group.

Specific examples of compounds in accordance with the invention are 1-methyl-2,6-diazabicyclo(2,2,2)octa-3,5-dione, 1,2,6-trimethyl-2,6-diazabicyclo(2,2,2)octa-3,5-dione, 1,4-dimethyl-2,6-diazabicyclo(2,2,2)octa-3,5-dione, 1-ethyl-2,6-diazabicyclo(2,2,2)octa-3,5-dione and 1,8-dimethyl-2,6-diazabicyclo(2,2,2)octa-3,5-dione.

These compounds can be used as starting products for the preparation of the corresponding octanes.

The invention further relates to a process for the preparation of a 2,6-diazabicyclo(2,2,2)octa-3,5-dione according to Formula I.

This process is characterized in that a compound with Formula II of the formula sheet, in which R and R' represent an alkyl

group or H and $R_1$-$R_6$ have the same meaning as in Formula I, is reacted with ammonia or a primary amine $R_2$-$NH_2$, in which formula $R_2$ has the same meaning as in Formula I. It is preferable to use an excess of the $NH_3$ or primary amine in relation to the compound of Formula II. The stoichiometric quantity of $NH_3$ or the primary amine is 2 moles per mole of the end product. It is of course possible to use a deficient quantity of the $NH_3$ or primary amine, but this is less advantageous.

The maximum quantity of the $NH_3$ or primary amine is essentially determined by the economy of the process.

The reaction with ammonia gives no substituents in positions 2 and 6, whilst the reaction with a primary amine gives an N-substituted compound.

An example of a starting compound is ethyl 2-carbethoxy-5-oxo-caproate. Upon reaction with ammonia this compound gives 1-methyl-2,6-diazabicyclo(2,2,2)octa-3,5-dione with liberation of ethanol.

In view of the nature of the reaction, R and R' should preferably be lower alkyl groups, such as methyl or ethyl.

The reaction may be carried out in accordance with known methods for the preparation of amides (J. March, Advanced Organic Chemistry: Reactions, Mechanisms, and Structure, McGraw-Hill Book Co., New York, 1968, pp. 338-339). The process may be carried out in a solvent inert with respect to the reaction product. However, the solvent may also be the primary amine that is to take part in the reaction.

It has been discovered that it is advantageous to use a solvent in which the starting compound is readily soluble and the reaction product is insoluble or only sparingly soluble, so that the reaction product is precipitated during the reaction. Examples of such solvents are water and lower alkanols ($C_{1-4}$) such as methanol, ethanol, and propan-1-ol.

It is generally unnecessary to add a catalyst to the reaction mixture. Under certain circumstances it may be advantageous to work at an elevated temperature and/or an elevated pressure to improve the reaction rate and/or the yield.

After termination of the reaction the product is separated from the reaction mixture, for example by distilling the solvent off or by filtering the solids off. The solids can then be washed and dried. If

necessary, the diazabicyclooctadione obtained may be recrystallized one or more times.

If the starting compound is an ester of Formula II, the alkanol formed in the reaction may be recovered and recycled.

The compound of Formula II may be prepared by any of the known methods for such compounds, for example by the reaction of an α,β-unsaturated ketocompound (Formula III) with malonic acid, a malonic ester, or a derivative of these compounds (Formula IV).

In this way the reaction of butenone with diethyl malonate gives the above-mentioned ethyl 2-carbethoxy-5-oxocaproate.

The invention will now be further explained by means of individual examples. If a decomposition temperature is given in an example it means that the compound decomposed before reaching the melting point.

Example I

Preparation of 1-methyl-2,6-diazabicyclo(2,2,2)octa-3,5-dione

230 g of ethyl 2-carbethoxy-5-oxocaproate and 270 ml of 25 wt-% aqueous ammonia were mixed at room temperature in a round-bottom flask fitted with a stirrer, thermometer, and reflux condenser.

The temperature rose slowly to 34°C as the ester dissolved. Solids started to crystallize out after about 1 h. About 5 h after the start of the reaction the temperature began to fall. By about 16 h after the start of the reaction the temperature had fallen to 20°C; at this stage the solids were filtered off and washed with water. After drying, 106 g of a compound that was subsequently identified as 1-methyl-2,6-diazabicyclo(2,2,2)octa-3,5-dione was obtained. The yield was thus 68.8%. The compound decomposed at 250°C.

Identification of 1-methyl-2,6-diazabicyclo(2,2,2)octa-3,5-dione

a. Mass spectrometry

The mass spectrum of the compound was recorded with AEI instruments $MS_9$ and $MS_{30}$.

The mass spectrum showed a weak peak at m/e 154.

The empirical formula of the strong fragment of mass 126 was $C_5H_6N_2O_2$ calculated from an accurate mass measurement at a resolving power of about 15000. This means that $C_2H_4$ had split off, which indicates the presence of a $C_2$ bridge.

b. NMR spectrometry

The $^1H$- and $^{13}C$-NMR spectra of the compound were determined with an XL-100A Varian spectrometer at room temperature, using deuterated formic acid as solvent and TMS as reference. The results were as follows:

$^1H$ NMR : δ = 1.73 (s, 3, $CH_3$)
δ = 2.10 (m, 4, $CH_2$)
δ = 3.60 (t, 1, CH)
δ = 11.16 (s, 2, NH)

$^{13}C$ NMR: δ = 19.37 ($C_9$, $^1J_{CH}$ = 28.5 Hz)
δ = 19.9 ($C_8$, $^1J_{CH}$ = 31.5 Hz)
δ = 33.05 ($C_7$, $^1J_{CH}$ = 30.0 Hz)
δ = 50.0 ($C_4$, $^1J_{CH}$ = 34 Hz)
δ = 68.4 ($C_1$, $^1J_{CH}$ = 0 Hz)
δ = 175.1 ($C_3$, $C_5$ $^1J_{CH}$ = 0 Hz)

Comparison of these spectra with those of the starting compounds showed that the product was 1-methyl-2,6-diazabicyclo(2,2,2)octa-3,5-dione.

In all the following examples the structure of the reaction product was established by $^{13}C$- or $^1H$-NMR.

Example II

Preparation of 1-methyl-2,6-diazabicyclo(2,2,2)octa-3,5-dione

In the same way as described in Example I, 103 g of 1-methyl-2,6-diazabicyclo(2,2,2)octa-3-dione was obtained from 202 g of methyl 2-carbomethoxy-5-oxocaproate and 270 ml of a 25 wt-% aqueous solution of ammonia after 16 h (yield 66.9%). The compound decomposed at 250°C. Identification took place as described in Example I.

Example III

Preparation of 1,2,6-trimethyl-2,6-diazabicyclo(2,2,2)octa-3,5-dione

In the same way as in Example II, 24.5 g of 1,2,6-trimethyl-2,6-diazabicyclo(2,2,2)octa-3,5-dione was obtained from 38.0 g of methyl 2-carbomethoxy-5-oxocaproate and 80 ml of a 35% aqueous

solution of methylamine after evaporating the methylamine-water mixture and recrystallizing the resulting residue from propan-1-ol. The yield was 71.6%. The melting point was 162-163°C. Identification took place as described in Example I.

Example IV

Preparation of 1-ethyl-2,6-diazabicyclo(2,2,2)octa-3,5-dione

In the same way as in Example I, 4.1 g of 1-ethyl-2,6-diazabicyclo(2,2,2)octa-3,5-dione was prepared from 9.0 g of ethyl 2-carbethoxy-5-oxoenanthate and 20 ml of a 25 wt-% aqueous solution of $NH_3$. The yield was 66%. The compound had a melting point (after crystallization from ethanol) of 170-171 °C. Identification took place as described in Example I.

Example V

Preparation of 1,4-dimethyl-2,6-diazabicyclo(2,2,2)octa-3,5-dione

4.3 g of 1,4-dimethyl-2,6-diazabicyclo(2,2,2)octa-3,5-dione was prepared from 26 g of ethyl 2-methyl-2-carbethoxy-5-oxocaproate and 35 ml of a 25 wt-% aqueous solution of $NH_3$. The selectivity was 90%. The compound decomposed at 240°C. Identification took place as described in Example I.

Example VI

Preparation of 1,2,6,8-tetramethyl-2,6-diazabicyclo(2,2,2)-octa-3,5-dione

In the same way as described in Example III, 5.6 g of 1,2,6,8-tetramethyl-2,6-diazabicyclo(2,2,2)octa-3,5-dione was obtained from 10 g of ethyl 2-carbethoxy-3-methyl-5-oxocaproate with a yield of 70%. The melting point was 137°C. Identification took place as described in Example I.

Example VII

Preparation of 1,2,4,6-tetramethyl-2,6-diazabicyclo(2,2,2)-octa-3,5-dione

In the same way as in Example III, 5.6 g of 1,2,4,6-tetramethyl-2,6-diazabicyclo(2,2,2)octa-3,5-dione was obtained from 26 g of

0080245

ethyl 2-methyl-2-carbethoxy-5-oxocaproate and 40 ml of a 35 wt-% aqueous solution of methylamine. The selectivity was 69.5% . 16 g of crude starting product was also recovered. The reaction product melted at 165°C. Identification took place as described in Example I.

Example VIII

Preparation of 1,8-dimethyl-2,6-diazabicyclo(2,2,2)octa-3,5-dione

In the same way as in Example I, 6.35 g of 1,8-dimethyl-2,6-diazabicyclo(2,2,2)octa-3,5-dione was obtained from 15 g of ethyl 2-carbethoxy-3-methyl-5-oxocaproate and 30 ml of a 25 wt-% aqueous solution of ammonia. The yield was 63% and the compound decomposed at 240°C. Identification took place as described in Example I.

AE 3344
0080245

CLAIMS

1. Diazabicyclo(2,2,2)octadione characterized by Formula I of the formula sheet in which $R_1$-$R_6$ may be the same or different and represent H or a methyl or ethyl group.
2. Compound in accordance with Claim 1, characterized in that $R_1$ represents a methyl or ethyl group.
3. Compound in accordance with Claim 1, characterized in that $R_2$ represents a methyl or ethyl group.
4. Process for the preparation of a diazabicyclo(2,2,2)octadione according to Claim 1, characterized in that a compound with Formula II of the formula sheet, in which R and R' are H or an alkyl group and $R_1$-$R_6$ have the same meaning as in Formula I, is reacted with ammonia or a primary amine $R_2$-$NH_2$, in which formula $R_2$ has the same meaning as in Formula I.

0080245

FORMULA PAGE

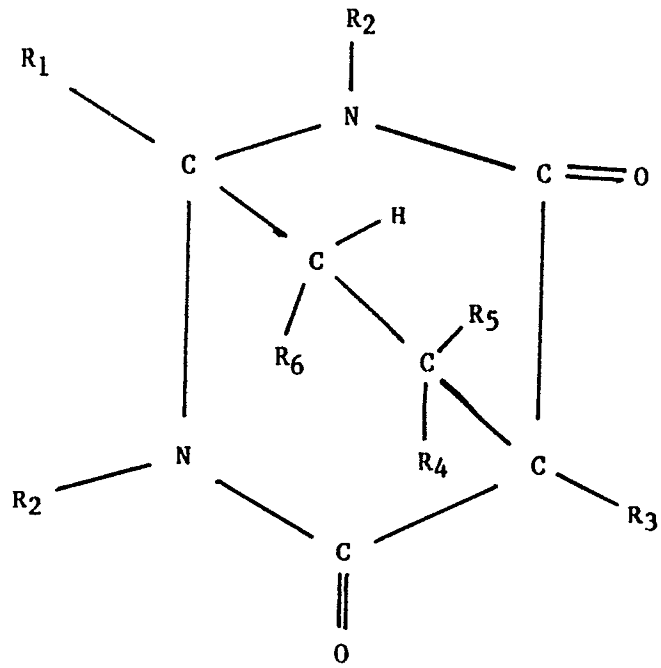

I.

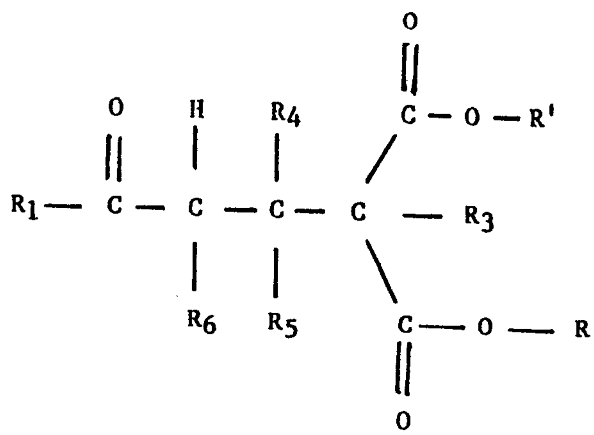

II.

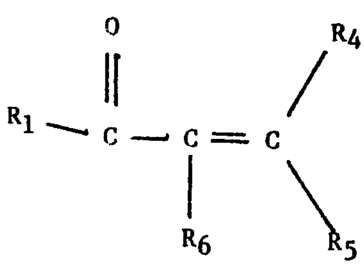

III.

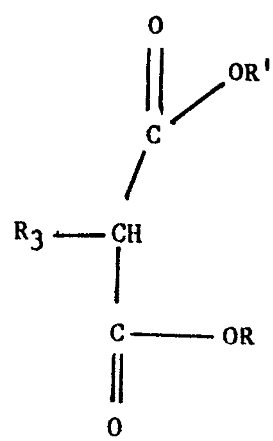

IV.

0080245

European Patent Office

# EUROPEAN SEARCH REPORT

Application number

EP 82 20 1484

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| A | JOURNAL OF ORGANIC CHEMISTRY, vol. 45, no. 14, 1980, pages 2754-63, Washington (USA); W.WYNBERG et al.: "Chiral tetraalkylmethanes. Two syntheses of optically active butylethylmethylpropylmethane of known and high optical purity". *Pages 2761-2, compound 29*<br>----- | 1 | C 07 D 471/08 //<br>(C 07 D 471/08<br>C 07 D 239/00<br>C 07 D 221/00 ) |
| | | | TECHNICAL FIELDS SEARCHED (Int. Cl. 3) |
| | | | C 07 D 471/00 |

The present search report has been drawn up for all claims

| Place of search | Date of completion of the search | Examiner |
|---|---|---|
| THE HAGUE | 25-02-1983 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82